# EUROPEAN PATENT SPECIFICATION

(11) **EP 2 044 853 B1**
(45) Date of publication and mention of the grant of the patent: **23.02.2011**
(21) Application number: 07117765.3
(22) Date of filing: 02.10.2007
(51) Int. Cl.: A23L 1/30, A23L 1/305, A23L 1/308, A23L 1/29, A61K 31/195, A23L 2/52, A61K 31/715, A61K 31/718

(54) **Nutriment for use for accelerated physiological performance enhancement**
Nährstoff zur Erhöhung beschleunigter physiologischer Leistung
Nutriment pour l'amélioration accélérée de la performance physiologique

(43) Date of publication of application: 08.04.2009
(73) Proprietor: ISME Privates Forschungsinstitut für Sport, Medizin und Ernährung GmbH, 64546 Mörfelden-Walldorf (DE)
(72) Inventor: Geiss, Kurt-Reiner, Dr, 63225 Langen (DE)
(74) Representative: Richardt, Markus Albert

(56) References cited:
- EP-A- 0 875 155
- WO-A-00/27408
- WO-A-2005/084323
- WO-A-2006/009437
- WO-A-2006/077202
- US-A1- 2002 197 352

## Description

### Field of the invention

The present invention relates to a nutriment in dry powder form and a liquid drink containing the nutriment.

### Background and related art

Many people are active in various kinds of sports. Typically, a sportsman prepares for a sports game by intensive workout. But the success of a sportsman during a game also strongly depends on a well balanced nutrition before a sports event, such that a high muscular effort is guaranteed during the sports game. Another important factor is the warm-up phase of players just before a game, which is necessary in order to enhance the blood circulation and in order to prevent injuries due to pulled muscles etc.

However, especially with respect to the warm-up phase, for sportsman participating in baseball, football, hockey, basketball, handball etc., a further problem exists: all the cited sports games have in common, that substitute players are used in order to allow substituting one player versus another player in game. Especially for sports games using substitutes, the challenge arises that due to for example an accident of an active player, this injured player has to be substituted by another player, which, since he was only designated as substitute player, has not previously in the game participated in a special warm-up exercise. Often, a decision for substitution of a player is made during halftime of the game when players are relaxing. In such situation the coach may make the decision to substitute one of the active players for various reasons. Even if a designated substitute player would have participated right before the game in a warm-up exercise, for example after 50 minutes waiting on the substitute's bench he will have cooled down completely.

This leads to the challenge that in order to prevent injuries due to for example pulled muscles, the substitute player needs to be given the possibility to warm-up extremely quickly. Further, a high physiological performance has to be guaranteed when the player gets into the game.

With respect to the physiological performance, for example, US 2002/0197352 A1 discloses a nutritional composition in a dry power form or a liquid drink for optimizing muscle performance during exercise.

WO 00/27408 discloses a composition for optimizing muscle performance during exercise.

DE 19903560 discloses a composition for improving performance at sports activities, its production and use as dietetic food product.

WO 2005/084323 does disclose a composition of bioactive compounds comprising amino acids including arginine and ornithine with carbohydrates, proteins, fat, vitamins and minerals. Optionally, the compounds are from fenugreek. According to one preferred embodiment a composition of bioactive compounds for enhancing the transport of glucose into muscle cells is provided.

WO 2006/009437 does disclose the use of specific protein and/or peptide fractions having high aspartate content for regulating plasma glucose concentrations and increasing insulin sensitivity in a mammal. A suggested nutritional product may comprise for example ornithine, proteins, fat, vitamins, minerals and different kinds of carbohydrates like maltodextrins, fructose, galactose, glucose and other sugars.

### Summary of the invention

The present invention provides a nutriment in dry powder form comprising L-Arginine in the range of 0.5% to 3%, preferably 1.0% to 1.6% by weight of said dry powder and L-Ornithine in the range of 0.5% to 3%, preferably 1.0% to 1.6% by weight of said dry power, the nutriment further comprising carbohydrates in the range of 70% to 95%, preferably 82% to 94% by weight of said dry powder. Said carbohydrates comprise a first kind of carbohydrates in a fraction of 45% to 65%, preferably 55% to 60% by weight of said carbohydrates and with a Glycaemia index of 60 to 80, in particular 65 to 75, preferably 70. The carbohydrates further comprise a second kind of carbohydrates in a fraction of 25% to 40%, in particular 30% to 35% by weight of said carbohydrates and with a Glycaemia index of 90 to 110, in particular 95 to 105, preferably 100. Said carbohydrates further comprise a third kind of carbohydrates in a fraction of 3% to 17%, in particular 5% to 10% by weight of said carbohydrates with a Glycaemia index higher than 125, preferably 136, wherein the first kind of carbohydrates comprises starch, the second kind of carbohydrates comprises maltodextrin and the third kind of carbohydrates comprises dextrose.

The nutriment according to the invention has the advantage, that due to the usage of L-Arginine and L-Ornithine a vascular dilatation is attained. Optimal results can be attained using 1.3% by weight of each of L-Arginine and L-Ornithine. Therewith, the blood circulation is enhanced, which significantly reduces the warm-up time of a sportsman. Also, the supply of muscles with carbohydrates is enhanced due to the increased blood circulation.

By using the combination of the three kinds of carbohydrates, a fast and continuous provision with carbohydrates is guaranteed such that the blood sugar level remains on optimal level independent on exercise, endurance and/or intensity.

Hence, by means of the nutriment according to the invention the warm-up time of a sportsman is reduced while the physiological performance is enhanced. Hence, the nutriment is for use for accelerated warm-up and prolonged physical performance.

In accordance with an embodiment of the invention, the nutriment further comprises proteins in the range of 1% to 14%, preferably 2% to 8% by weight. The usage of proteins in this range has the advantage, that carbohydrate and fat metabolism are affected in a positive manner. Also, the supplied proteins directly supply a part of the energy needs during a workout. Proteins stimulate the simultaneous release of growth hormones and insulin. The combined hormonal influence redirects dietary carbohydrate and fat to the aerobic muscle fibers where they are used for exhausting workouts.

Since proteins are also transport proteins, they are also responsible and necessary to transport important substances like hemoglobin, which is responsible in the blood for oxygen transport, to the muscles. Therewith, the supplied proteins have a supportive effect on the effective conversion of the supplied carbohydrates into energy necessary for usage by the muscles.

In accordance with an embodiment of the invention, the nutriment further comprises minerals in the range of 1 % to 3% by weight of said dry powder. Thereby, said minerals comprise sodium in a fraction of 60% to 75% by weight of said minerals, preferably 76%. Also, said minerals may comprise potassium in a fraction of 20% to 34% by weight of said minerals, preferably 27%. Further, said minerals may also comprise magnesium in a fraction of 2% to 8% by weight of said minerals, preferably 5%.

The usage of these minerals has several advantages. The first advantage is, that due to the well-balanced mineral selection a hypotonic nutriment can be provided, which means that a fast resorption in the body is provided. Therewith, the supplied L-arginine, L-ornithine and the carbohydrates can be resorbed by the body in an even more accelerated manner, which further accelerates the physiological performance enhancement.

In accordance with the invention, the first kind of carbohydrates comprises starch, the second kind of carbohydrates comprises maltodextrin and the third kind of carbohydrates comprises dextrose. These three kinds of carbohydrates have the advantage, that in the optimal combination indicated above a continuous and optimal blood sugar level can be attained which lasts without significant variations over a long period of time. Thereby, dextrose is resorbed by the body extremely quickly, wherein the resorption time of maltodextrin is in the intermediate range. Starch is resorbed and converted by the body relatively slow, so that starch is able to provide a high blood sugar level a relatively late point in time with respect to the time of digestion.

In accordance with an embodiment of the invention, the nutriment further comprises vitamins in the range of 0.0003% to 0.0008% by weight of said dry powder, wherein the vitamins comprise vitamin B1 and/or vitamin B2.

The addition of said vitamins to the nutriment has the advantage, that the process of converting carbohydrates to energy is further supported. Said vitamins play a crucial role in certain metabolic reactions, particularly the conversion of carbohydrates into sugar which is the energy source for the muscles. This means, that by addition of said vitamins muscle endurance is further increased. The addition of vitamin B2 is especially important, since the body does not store vitamin B2 so that a deficiency can happen more readily.

In accordance with an embodiment of the invention, a fat content of the nutriment is below 1 % by weight of said dry powder, preferably below 0.1 %. Using a nutriment with such a low fat level, preferably even without any fat has the advantage, that the effect of delayed carbohydrate resorption by the body due to the presence of fat can be avoided. Therewith, the accelerated physiological performance enhancement obtained by the nutriment according to the invention is maintained. Also, a "no fat" nutriment has psychological effects since people are getting more and more health conscious and try to avoid food and drinks containing fat. This holds especially for people doing sports. Therefore, a "no fat" nutriment will have a high acceptance by people.

In another aspect the invention relates to a liquid drink containing the nutriment according to the invention, wherein the nutriment is contained in the range of 5% to 10% by weight of said liquid drink, the liquid drink being for use for accelerated physiological performance enhancement. Thereby, the serving size is in between 400ml to 600ml. The liquid drink may for example contain water or milk or fruit juice as liquid basis.

Using a liquid drink has the advantage, that for example an athlete is able to easily digest the nutriment according to the invention. Stirring of the nutriment in powder form into for example water is not necessary, which is an important aspect for people which like to digest the nutriment for example in game, on the way, also immediately in the moment when they are substituted in game. Additionally, with the serving size in between 400ml to 600ml, an optimal amount of carbohydrates can be supplied which yields a substantially constant blood sugar level over 100 mg/dl over 60 to 90 minutes.

### Brief description of the drawings

In the following embodiments of the invention will be described in greater detail by way of example only making reference to the drawings in which:
- Figure 1: is a graph illustrating the impact of the nutriment according to the invention on physiological performance,
- Figure 2: is a graph illustrating the endurance time on highest intensity level using the nutriment according to the invention.
- Figure 3: is a table illustrating the ingredients of a drink according to the invention.

### Detailed description

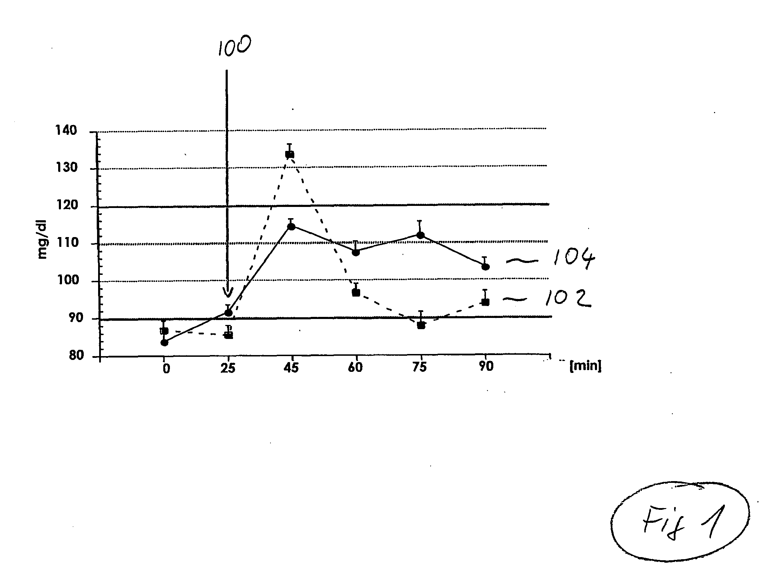

Fig. 1 is a graph illustrating the impact of the nutriment according to the invention on physiological performance. The graph shows the results of a clinical study. The abscissa is given in minutes, whereas the ordinate is given in mg/dl (dl = deciliter, 1 dl = 0,1 liter). The ordinate indicates thereby the blood sugar level depending on the time after which various kinds of carbohydrates have been supplied to a person. At a time of 25 minutes (reference numeral 100) carbohydrates have been supplied. Thereby, the curve 102 corresponds to a nutriment with a weighted mean Glycaemia index of 136, whereas the curve 104 corresponds to a nutriment comprising the carbohydrates according to the invention in combination with L-Arginine and L-Ornithine. Thereby, the average Glycaemia index is around 92.

As can be clearly seen, for the curve 102 a fast increase after supply of the nutriment after 25 minutes is attained. The blood sugar level is increasing relatively fast within 20 minutes to a maximum of 135 mg/dl, and thereafter suddenly dropping within 15 minutes to a blood sugar level below 100 mg/dl.

In contrast, with the nutriment according to the invention, the blood sugar level is increasing relatively fast to a level around 110 mg/dl, which level is reached after 20 minutes. This blood sugar level of 110 mg/dl thereafter remains on an optimal plateau kind level around 110 mg/dl independent on exercise, endurance and/or intensity. In other words, by applying the nutriment according to the invention, a constant supply in energy over a long period of time, in fig. 1 over 60 to 90 minutes, at least 75 minutes, can be attained. Thereby, the blood sugar level remains at least above 100 mg/dl.

Referring to the example of a sportsman which has to warm-up during halftime of a game, by using the nutrition according to the invention, a highly accelerated uptake of carbohydrates is achieved which results in an accelerated physiological performance already after the first few minutes after digestion. The combinatory presence of L-Arginine and L-Ornithine increases the blood circulation and especially the carbohydrates with high Glycaemia index are supplied in a highly accelerated manner to the muscles.

When the sportsman is in game, due to the well-balanced combination of various carbohydrates a continuous physiological performance enhancement corresponding to a continuous high blood level is obtained.

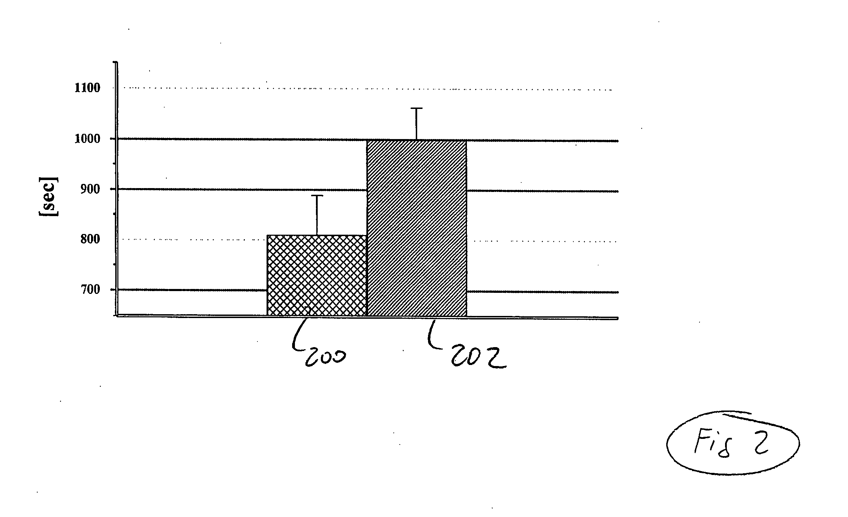

Fig. 2 is a graph illustrating the endurance time on highest intensity level using the nutriment according to the invention. Thereby, the ordinate in fig. 2 shows the endurance time, whereas on the abscissa the two kinds of carbohydrate solutions indicated in fig. 1 with the curves 102 and 104 are depicted. Thereby, the area 200 corresponds to the carbohydrate solution with the high Glycaemia index of 136 (reference numeral 102 in fig. 1), whereas the box 202 corresponds to the carbohydrate solution according to the invention with the Glycaemia index of 92 (curve 104 in fig. 1).

As can be clearly seen in fig. 2, a significantly prolonged endurance time on highest intensity level can be obtained applying the nutriment according to the invention. By using the carbohydrate solution with the high Glycaemia index, the endurance time on highest intensity level is limited to a maximum of 800 seconds. In contrast, using the nutrition according to the invention, an endurance time of about 1000 seconds is attained, which corresponds to a longer endurance time of about 20%. Therewith, a continuous exercising is possible.

It has to be annotated, that the nutrition according to the invention can also be provided to the body additionally during the exercise itself. This is especially important for endurance athletes, like marathon runners, which require energy in form of carbohydrates over a long period of time up to several hours. However, in general preferably 1 to 2 liquid drinks according to the invention, with a serving size in between 400g and 600g should be supplied to an athlete, preferably before and during the exercise.

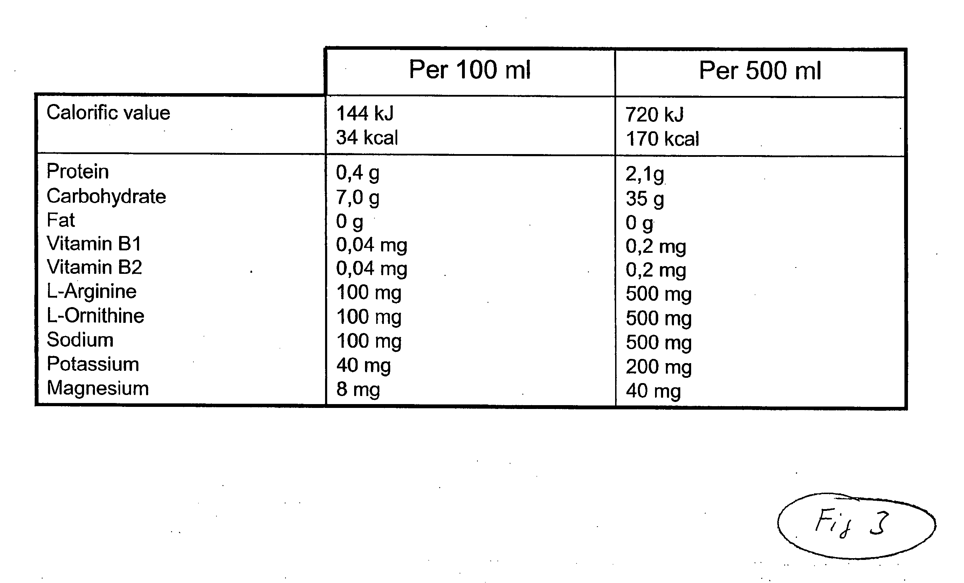

Figure 3 is a table illustrating the ingredients of a drink according to the invention. As can be seen, a typical drink contains the nutriment according to the invention such, that 100 ml drink comprise 0,4 g protein, 7,0 g carbohydrates, 0,04 mg Vitamin B1 and 0,04 mg Vitamin B2, each 100 mg of L-Arginine, L-Ornithine and Sodium, 40 mg of Potassium and 8 mg of Magnesium. With an optimal serving size of 500 ml, a sportsman is provided in a fast and continuous manner with carbohydrates such that the blood sugar level remains on optimal level independent on exercise, endurance and/or intensity.

## Claims

1. Nutriment in dry powder form comprising L-Arginine in the range of 0.5% to 3%, preferably 1.0% to 1.6% by weight of said dry powder and L-Ornithine in the range of 0.5% to 3%, preferably 1.0% to 1.6% by weight of said dry powder, the nutriment further comprising Carbohydrates in the range of 70% to 95% by weight of said dry powder, wherein said carbohydrates comprise
- a first kind of carbohydrates in a fraction of 45% to 65% by weight of said carbohydrates and with a Glycaemia index of 60 to 80, preferably 70,
- a second kind of carbohydrates in a fraction of 25% to 40% by weight of said carbohydrates and with a Glycaemia index of 90 to 110, preferably 100,
- a third kind of carbohydrates in a fraction of 3% to 17% by weight of said carbohydrates and with a Glycaemia index higher than 125, preferably 136
wherein
- the first kind of carbohydrates comprises starch,
- the second kind of carbohydrates comprises maltodextrin and
- the third kind of carbohydrates comprises dextrose.

2. The nutriment according to claim 1, further comprising proteins in the range of 1% to 14% by weight.

3. The nutriment according to any of the previous claims, further comprising minerals in the range of 1% to 5% by weight of said dry powder.

4. The nutriment according to claim 3, wherein said minerals comprise sodium in a fraction of 60% to 75% by weight of said minerals, preferably 67%.

5. The nutriment according to claim 3 or 4, wherein said minerals comprise potassium in a fraction of 20% to 34% by weight of said minerals, preferably 27%.

6. The nutriment according to any of the previous claims 3 to 5, wherein said minerals comprise magnesium in a fraction of 2% to 8% by weight of said minerals, preferably 5%.

7. The nutriment according to any of the previous claims, further comprising vitamins in the range of 0.0003% to 0.0008% by weight of said dry powder, wherein the vitamins comprise vitamin B1 and/or vitamin B2.

8. The nutriment according to any of the previous claims, wherein a fat content is below 1% by weight of said dry powder, preferably below 0.1 %.

9. The nutriment according to any of the previous claims, wherein the nutriment is for use for accelerated warm-up and prolonged physical performance.

10. A liquid drink comprising the nutriment according to any of the previous claims, wherein the nutriment is contained in the range of 5% to 10% by weight of said liquid drink.

11. The drink according to claim 10, wherein the serving size is in between 400ml and 600ml, preferably 500ml.

## Patentansprüche

1. Nährmittel in Form trockenen Pulvers mit L-Arginin im Bereich zwischen 0,5 und 3 %, vorzugsweise 1-1,6 %, des Gewichtes des Trockenpulvers und L-Ornithin im Bereich zwischen 0,5 und 3 %, vorzugsweise 1-1,6 %, des Gewichtes des Trockenpulvers, wobei das Nährmittel ferner Carbohydrate im Bereich zwischen 70 und 95 Gewichtsprozent des Trockenpulvers umfasst, wobei besagte Carbohydrate
- eine erste Art von Carbohydraten zu einem Bruchteil zwischen 45 und 65 Gewichtsprozent besagter Carbohydrate und mit einem Glycaemia-Index von 60-80, vorzugsweise 70,
- eine zweite Art von Carbohydraten zu einem Bruchteil zwischen 25 und 40 Gewichtsprozent der Carbohydrate und mit einem Glycaemia-Index von 90-110, vorzugsweise 100,
- eine dritte Art von Carbohydraten zu einem Bruchteil zwischen 3 und 17 Gewichtsprozent besagter Carbohydrate und mit einem Glycaemia-Index höher als 125, vorzugsweise 136,
umfassen, wobei
die erste Art von Carbohydraten Speisestärke umfasst,
die zweite Art von Carbohydraten Maltodextrin umfasst, und
die dritte Art von Carbohydraten Dextrose umfasst.

2. Nährmittel nach Anspruch 1, wobei das Nährmittel ferner Proteine im Bereich zwischen 1 und 14 Gewichtsprozent umfasst.

3. Nährmittel nach einem der vorhergehenden Ansprüche, wobei das Nährmittel ferner Mineralien im Bereich zwischen 1 und 5 Gewichtsprozent des Trockenpulvers umfasst.

4. Nährmittel nach Anspruch 3, wobei besagte Mineralien Natrium zu einem Bruchteil von 60-75 Gewichtsprozent der Mineralien, vorzugsweise 67 %, umfassen.

5. Nährmittel nach Anspruch 3 oder 4, wobei die Mineralien Kalium zu einem Bruchteil von 20-34 Gewichtsprozent der Mineralien, vorzugsweise 27 %, umfassen.

6. Nährmittel nach einem der vorhergehenden Ansprüche 3-5, wobei die Mineralien Magnesium zu einem Bruchteil von 2-8 Gewichtsprozent, vorzugsweise 5 %, der Mineralien umfassen.

7. Nährmittel nach einem der vorhergehenden Ansprüche, wobei das Nährmittel ferner Vitamine im Bereich zwischen 0,0003 bis 0,0008 Gewichtsprozent des Trockenpulvers umfasst, wobei die Vitamine Vitamin B1 und/oder Vitamin B2 umfassen.

8. Nährmittel nach einem der vorhergehenden Ansprüche, wobei der Fettgehalt weniger als 1 Gewichtsprozent des Trockenpulvers, vorzugsweise weniger als 0,1 Gewichtsprozent, ist.

9. Nährmittel nach einem der vorhergehenden Ansprüche, wobei das Nährmittel für ein schnelleres Aufwärmen und eine verlängerte körperliche Belastung ausgebildet ist.

10. Ein flüssiges Getränk, das das Nährmittel nach einem der vorhergehenden Ansprüche umfasst, wobei das Nährmittel in einem Bereich von 5-10 Gewichtsprozent des flüssigen Getränks enthalten ist.

11. Getränk nach Anspruch 10, wobei die Portionsgröße zwischen 400 und 600 ml, vorzugsweise 500 ml, beträgt.

## Revendications

1. Nutriments sous forme de poudre sèche comprenant de la L-arginine dans la gamme de 0,5% à 3%, de préférence de 1,0% à 1,6% en poids de ladite poudre sèche et de la L-ornithine dans la gamme de 0,5% à 3%, de préférence de 1,0% à 1,6% en poids de ladite poudre sèche, le nutriment comprenant en outre des glucides dans la gamme de 70% à 95% en poids de ladite poudre sèche, lesdits glucides comprenant
- un premier type de glucides dans une fraction de 45% à 65% en poids desdits glucides et ayant un indexe glycémique de 60 à 80, de préférence de 70,
- un deuxième type de glucides dans une fraction de 25% à 40% en poids desdits glucides et ayant un indexe glycémique de 90 à 110, de préférence de 100,
- un troisième type de glucides dans une fraction de 3% à 17% en poids desdits glucides et ayant un indexe glycémique supérieur à 125, de préférence de 136,
où
- le premier type de glucides comprend de l'amidon,
- le deuxième type de glucides comprend de la maltodextrine, et
- le troisième type de glucides comprend du dextrose.

2. Nutriment selon la revendication 1 comprenant en outre des protéines dans la gamme de 1% à 14% en poids.

3. Nutriment selon l'une quelconque des revendications précédentes, comprenant en outre des minéraux dans la gamme de 1% à 5% en poids de ladite poudre sèche.

4. Nutriment selon la revendication 3, **caractérisé en ce que** lesdits minéraux comprennent du sodium dans une fraction de 60% à 75% en poids desdits minéraux, de préférence de 67%.

5. Nutriment selon la revendication 3 ou 4, **caractérisé en ce que** lesdits minéraux comprennent du potassium dans une fraction de 20% à 34% en poids desdits minéraux, de préférence de 27%.

6. Nutriment selon l'une quelconque des revendications 3 à 5 précédentes, **caractérisé en ce que** lesdits minéraux comprennent du magnésium dans une fraction de 2% à 8% en poids desdits minéraux, de préférence de 5%.

7. Nutriment selon l'une quelconque des revendications précédentes comprenant en outre des vitamines dans la gamme de 0,0003% à 0,0008% en poids de ladite poudre sèche, lesdites vitamines comprenant de la vitamine B1 et/ou de la vitamine B2.

8. Nutriment selon l'une quelconque des revendications précédentes **caractérisé en ce que** la teneur en graisses est inférieure à 1% en poids de ladite poudre sèche, de préférence inférieure à 0,1%.

9. Nutriment selon l'une quelconque des revendications précédentes, **caractérisé en ce que** le nutriment est destiné à être utilisé pour l'échauffement accéléré et les performances physiques prolongées.

10. Boisson liquide comprenant le nutriment selon l'une quelconque des revendications précédentes, **caractérisée en ce que** le nutriment est compris dans la gamme de 5% à 10% en poids de ladite boisson liquide.

11. Boisson selon la revendication 10, **caractérisée en ce que** la portion à servir est comprise entre 400 ml et 600 ml, de préférence de 500 ml.
